(19) Europäisches Patentamt
European Patent Office
Office européen des brevets

(11) Numéro de publication : **0 308 349 B1**

# (12) FASCICULE DE BREVET EUROPEEN

(45) Date de publication du fascicule du brevet : **11.12.91 Bulletin 91/50**

(51) Int. Cl.[5] : **C07C 303/00**, C07C 215/00, A61K 31/14, A61K 31/325

(21) Numéro de dépôt : **88402352.4**

(22) Date de dépôt : **16.09.88**

## (54) Nouveau derivé de choline, son procédé de préparation et nouveaux médicaments le contenant.

(30) Priorité : **18.09.87 FR 8712968**

(43) Date de publication de la demande : **22.03.89 Bulletin 89/12**

(45) Mention de la délivrance du brevet : **11.12.91 Bulletin 91/50**

(84) Etats contractants désignés : **AT BE CH DE ES FR GB GR IT LI LU NL SE**

(56) Documents cités :
EP-A- 0 179 694
CH-A- 488 678
DE-B- 2 445 679

(73) Titulaire : **LAFON PHARMA S.A.**
**14 Boulevard de Perolles**
**CH-17000 Fribourg (CH)**

(72) Inventeur : **Nguyen, Dat Xuong**
**93 Rue Roger Salengro**
**F-92160 Antony (FR)**
Inventeur : **Rapin, Jean**
**15 Square de Châtillon**
**F-75014 Paris (FR)**
Inventeur : **Staron, Thadée**
**14 Résidence "L'Orée de Marly"**
**F-78590 Noisy le Roi (FR)**

(74) Mandataire : **Le Guen, Gérard et al**
**CABINET LAVOIX 2, place d'Estienne d'Orves**
**F-75441 Paris Cédex 09 (FR)**

EP 0 308 349 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un nouveau dérivé de l'acide diéthyl-dithio-carbamique, le diéthyl-dithio-carbamate de choline, son procédé de préparation, et son utilisation thérapeutique.

Le nouveau composé selon l'invention, le diéthyl-dithio-carbamate de choline, de formule brute $C_{10}$ $H_{24}$ $N_2$ O $S_2$, répond à la formule développée suivante :

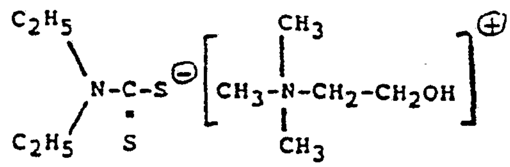

et a un poids moléculaire de 252,40.

Ce nouveau sel de choline se présente sous forme de fins cristaux incolores ou légèrement jaunâtres, ayant un point de fusion à 96-97°C, facilement solubles dans l'eau, dans les alcools et les glycols, et beaucoup moins solubles dans les solvants organiques.

Les cristaux de diéthyl-dithio-carbamate de choline sont très hygroscopiques et doivent donc être conservés dans une enceinte hermétique et froide.

| | Calculé | Trouvé | Moyenne |
|---|---|---|---|
| Garbone | 48,85 % | 48,18 - 48,47 | 48,33 % |
| Hydrogène | 9,64 % | 9,37 - 9,54 | 9,45 % |
| Azote | 11,12 % | 11,59 - 11,51 | 11,55 % |
| Soufre | 25,55 % | 24,94 - 24,75 | 24,85 % |
| Oxygène | 6,35 % | | |

Les spectres I.R.,U.V.,R.M.N. et de masse montrent bien les bandes caractéristiques de la molécule du nouveau produit de l'invention.

Le diéthyl-dithio-carbamate de choline peut être préparé par synthèse à partir du diéthyl-dithio-carbamate de sodium tri-hydraté et du chlorure de choline.

La réaction est la suivante :

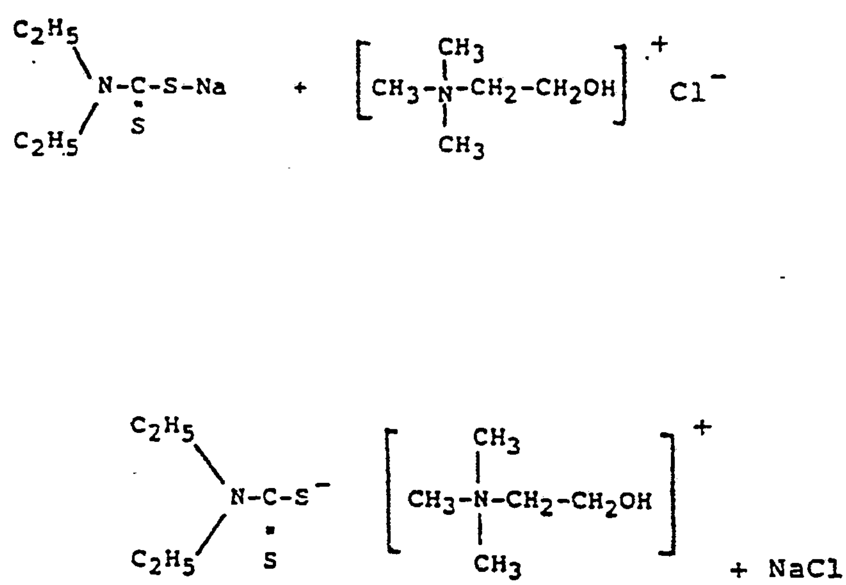

La synthèse peut être opérée selon le mode opératoire suivant :

Dans deux erlens ou 2 bechers de capacité appropriée, on dissout séparément :

— 56 g (0,40 mole) de chlorure de choline dans 100 ml d'éthanol absolu en le faisant tiédir à l'aide d'une plaque électrique ou d'un bain-marie et en remuant la solution pour favoriser la dissolution.

— 90 g (0,40 mole) de diéthyl-dithio-carbamate de sodium tri-hydraté dans 100 ml d'éthanol absolu.

Quand les deux solutions sont limpides, on introduit lentement en mince filet la solution alcoolique de choline dans celle du sel sodique, ceci sous forte agitation et en refroidissant extérieurement par un bain d'eau froide, car la réaction est très exothermique.

Quand le tiers environ est ajouté, il se forme un abondant précipité. On continue l'addition tout en maintenant une très vigoureuse agitation.

L'addition de la solution alcoolique de choline dans celle du sel sodique dure environ 15 minutes. On continue l'agitation encore pendant 30 minutes. On laisse ensuite refroidir pendant toute une nuit. Le récipient contenant la masse réactionnelle restant fermé.

Le lendemain on élimine le chlorure de sodium par essorage et on lave sur filtre deux fois avec 50 ml d'éthanol absolu.

On récupère environ 23 g de chlorure de sodium.

On concentre les eaux-mères alcooliques (contenant le diéthyl-dithio-carbamate de choline dissous dans l'éthanol) au bain-marie, sous le vide de la trompe-à-eau pour chasser tout l'excès d'éthanol.

On verse ensuite rapidement le résidu très visqueux dans un becher ou un mortier en verre ou en porcelaine. On sèche dans un dessicateur sous bon vide avec le pentoxyde de phosphore comme agent desséchant.

Le produit obtenu se présente en bloc que l'on réduit en fins cristaux ou en fine poudre micro-cristalline qui est incolore ou très légèrement jaunâtre.

Le rendement quantitatif en diéthyl-dithio-carbamate de choline obtenu est d'environ 100 g.

Le diéthyl-dithio-carbamate de choline ainsi obtenu doit être conservé dans une enceinte hermétique à cause de son hygroscopie.

Selon la présente invention, on a mis en évidence les propriétés thérapeutiques du nouveau sel de choline et on a fait une étude très complète de sa toxicité.

L'étude toxicologique a été effectuée sur la souris blanche SWISS.

Chez cet animal on a pu étudier pour le diéthyl-dithio-carbamate de choline, la toxicité aiguë, la toxicité chronique, l'influence sur la prolificité, la tératogénèse, la fertilité dans le temps et la longévité, l'influence sur l'efficacité de la réponse immunitaire.

Pour toutes ces expériences, les animaux ont été randomisés, séparés selon les sexes, répartis par lots de 30, les femelles gravides étant isolées en cages individuelles. Ces animaux étaient nourris avec un aliment complet convenable qu'ils pouvaient consommer ad libitum, pour éviter toute carence au cours de l'expérimentation. Leur boisson était l'eau ordinaire additionnée ou non du produit attesté, à savoir le diéthyl-dithio-carbamate de choline.

L'aliment complet utilisé contenait des céréales, des protéines végétales, des protéines animales, des sels minéraux et des vitamines.

La mesure de la toxicité aiguë du nouveau composé selon l'invention, administré par voie intrapéritonéale et sous-cutanée, montre que cette toxicité aiguë est très faible :

Au-dessous de 1 g/kg il n'y a pas de mortalité.

La DL 50 est comprise entre 1,4 et 1,7 g/kg.

Le graphique suivant illustre ces résultats.

TOXICITE AIGUË CHEZ LA SOURIS EN G/KG

(voie intrapéritonéale et sous-cutanée)

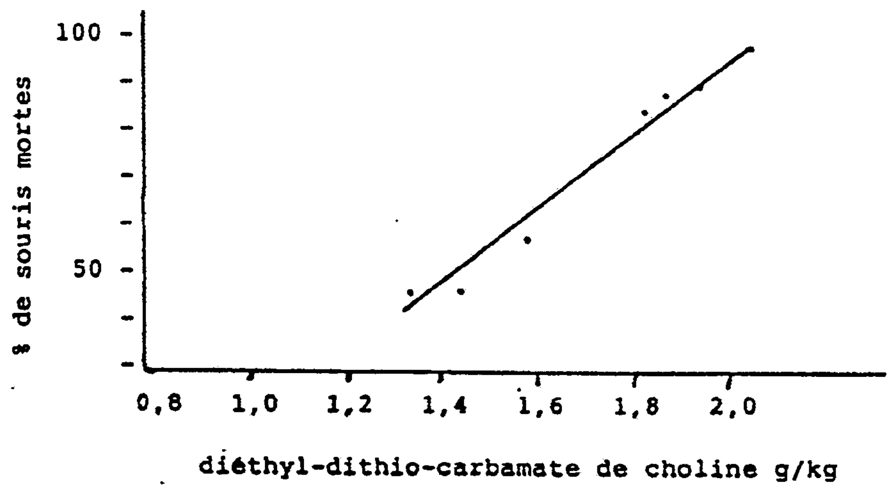


Pour mesurer la toxicité chronique du nouveau composé selon l'invention, on a administré par voie buccale dans l'eau de boisson une dose de 20 mg/kg par jour du nouveau composé, à 4 lots de 30 souris pendant 100 jours. Aucun effet néfaste sur la santé des animaux n'a pu être relevé.

On a examiné quel était l'effet du nouveau composé selon l'invention sur la prolificité et la tératogénèse des souris blanches SWISS. Les expériences ont été poursuivies sur quatre générations d'animaux et ont montré que les lots recevant le nouveau composé selon l'invention avaient globalement 15% de plus de descendants que les lots témoins, ceci avec un taux de souriceaux mort-nés inférieur de moitié.

Les résultats figurent dans le tableau ci-dessous :

EXPERIENCES DE PROLIFICITE ET DE TETRATOGENESE EFFECTUEES SUR QUATRE GENERATIONS DE SOURIS BLANCHES FEMELLES SWISS DE 4 MOIS

(Lots T - témoins; lots G - diéthyl-dithio-carbamate de choline à 1 °/•• dans l'eau de boisson)

| Générations | Nombre de souris gravides contrôlées à chaque génération | | Nombre de souriceaux nés vivants | | Nombre de souriceaux mort-nés | | Nombre de foetus résorbés | |
|---|---|---|---|---|---|---|---|---|
| | T | G | T | G | T | G | T | G |
| I | 30 | 30 | 255 | 249 | 7 | - | 15 | 2 |
| II | 30 | 30 | 242 | 316 | 5 | 2 | 9 | 2 |
| III | 30 | 30 | 258 | 324 | 8 | - | 8 | - |
| IV | 30 | 30 | 263 | 308 | 3 | - | 11 | - |
| Totaux | 120 | 120 | 1018 | 1197 | 23 | 2 | 43 | 4 |

De même la mesure de la fertifilité des souris femelles en fonction de leur âge a montré que l'administration du nouveau composé selon l'invention allonge substantiellement la période de fertilité des souris femelles

comme le montre le tableau ci-dessous :

**EXPERIENCES DE FERTILITE DES SOURIS FEMELLES SWISS SELON LEUR AGE**

(Lots T = témoins; lots G = Diéthyl-dithio-carbamate de choline à 1 °/.. dans l'eau de boisson

| | % de souris femelles fertiles | | nombre moyen de souriceaux par souris fertile | |
|---|---|---|---|---|
| | T | G | T | G |
| souris de 4 mois | 97 | 97 | 8,5 | 9,8 |
| souris de 8 mois | 62 | 92 | 6,9 | 8,0 |
| souris de 15 mois | 23 | 60 | 5,2 | 7,5 |

Enfin, en ce qui concerne la croissance et la longévité des animaux, on a pu remarquer que le nouveau composé selon l'invention n'avait pas d'influence négative sur la croissance malgré une réduction d'environ 25% de la prise d'eau et d'environ de 5% de la prise de nourriture. On a au contraire constaté que l'administration du nouveau composé selon l'invention retardait considérablement le vieillissement. Cette dernière activité est particulièrement spectaculaire chez les souris mâles, et semble due à une modification des caractères et des comportements amenant une diminution de la vulnérabilité qui résulte habituellement d'une très forte agressivité. Les mâles témoins se battent beaucoup plus, s'épuisent, se blessent et se tuent.

EXPERIENCES DE LONGEVITE

COURBES DE MORTALITE DES SOURIS SWISS MALES ET FEMELLES abreuvées avec de l'eau ordinaire pour les témoins et de l'eau ordinaire additionnée de 1 °/.. de diéthyl-dithio-carbamate de choline pour les lots expérimentaux

(chaque courbe est la moyenne de 10 lots de 30 souris).

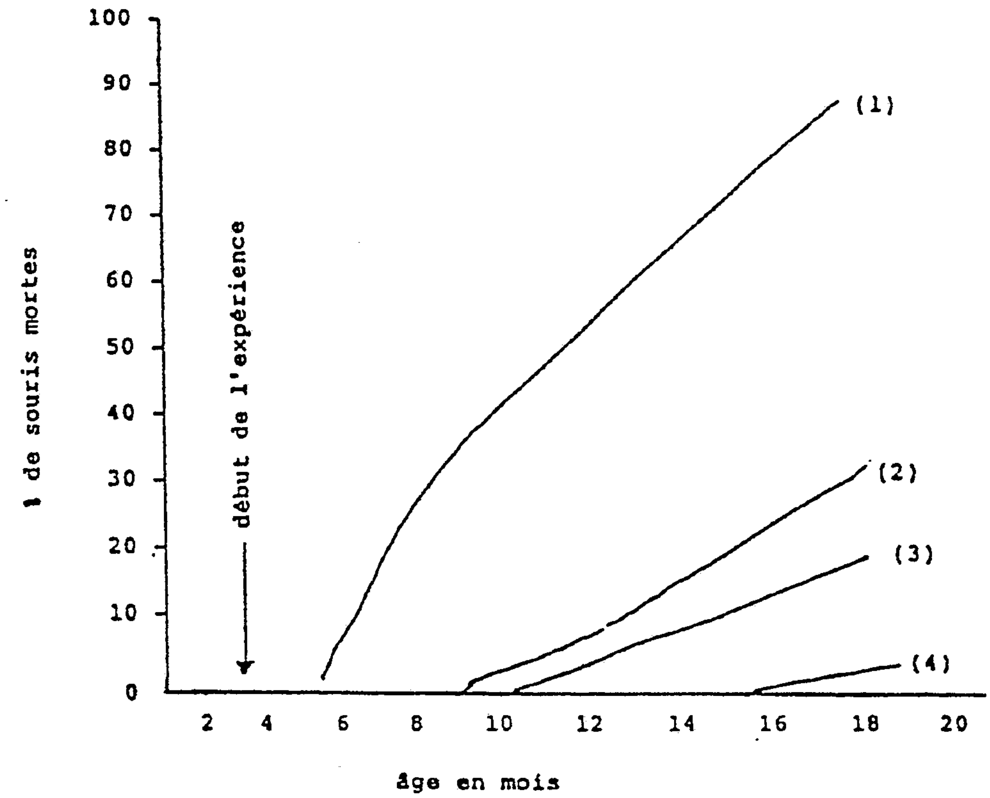


(1) souris mâles témoins
(2) souris mâles recevant du diéthyl-dithio-carbamate de choline
(3) souris femelles témoins
(4) souris femelles recevant du diéthyl-dithio-carbamate de choline

On a également étudié l'influence du nouveau composé selon l'invention sur l'efficacité de la réponse immunitaire chez les souris blanches SWISS.

La protection des animaux supérieurs contre les agressions est assurée par le système immunitaire en relation avec les antigènes du complexe majeur d'histocompatibilité. L'efficacité de la réaction immunitaire dépend des mécanismes et d'équilibres conjugués qui déterminent les principaux paramètres de la compétence (sensibilité, précision, intensité, durée). Ainsi, les multiples et diverses agressions sont habituellement rapidement maîtrisées sans être sciemment perçues par l'hôte agressé. Mais lorsque les réponses inappropriées et erronées sont fréquentes, elles sont à l'origine de nombreuses maladies inflammatoires difficiles à maîtriser et qui s'amplifient généralement avec le vieillissement. Dans ces cas là, une stratégie hygiénique et des interventions thérapeutiques s'imposent pour corriger les erreurs immunitaires, maîtriser l'inflammation et pour rétablir la fonctionnalité des tissus et des territoires lésés.

Le tableau ci-après illustre la potentialisation de la compétence immunitaire par le diéthyl-dithio-carbamate de choline. Le tableau montre en effet que pour obtenir la même protection immunitaire, il faut 100 mcg de

vaccin spécifique chez les témoins et seulement 20 mcg pour les souris traitées avec le composé selon l'invention.

**INFLUENCE DU DIETHYL-DITHIO-CARBAMATE DE CHOLINE CHEZ LA SOURIS, SUR L'EFFICACITE DE LA REPONSE IMMUNITAIRE A L'INJECTION SOUS-CUTANEE D'UN ESCHERICHIA COLI PATHOGENE**

(T = lots témoins; G = lots abreuvés depuis 21 j avec de l'eau de boisson comportant 1°/.. de diéthyl-dithio-carbamate de choline)

| | | Quantités de vaccin injectées par voie sous-cutanée (mcg/souris de 25 g), 8 jours avant l'épreuve pathogène | | | | | | | | | | | | | | | |
|---|---|---|---|---|---|---|---|---|---|---|---|---|---|---|---|---|---|
| | | 0 | | 3 | | 10 | | 20 | | 50 | | 70 | | 100 | | 150 | |
| | | T | G | T | G | T | G | T | G | T | G | T | G | T | G | T | G |
| Dose léthale (DL) en %, de l'inoculum pathogène, selon la dose de vaccin injectée | $DL_0$ | | | | | | | | + | | + | | + | + | + | + | + |
| | $DL_{30}$ | | | | | | + | | | | | | | | | | |
| | $DL_{50}$ | | | | | | | | | | | + | | | | | |
| | $DL_{70}$ | | | | + | | | + | | + | | | | | | | |
| | $DL_{100}$ | + | + | + | | + | | | | | | | | | | | |

Il résulte de toutes les expérimentations relatées ci-dessus que la toxicité du nouveau composé selon l'invention est pratiquement nulle. Il apparaît également que l'administration de ce nouveau sel de choline aux souris blanches SWISS entraîne des effets très favorables sur la prolificité, la longévité, les comportements et l'efficacité de la réponse immunitaire.

Les études pharmacologiques du nouveau composé selon l'invention ont montré qu'il possédait un effet chélatant des métaux lourds et de transition, un effet immuno-stimulant, un effet anti-dégénératif et un effet sur les transferts neuronaux, et qu'il présentait par conséquent une très intéressante activité thérapeutique dans le domaine des maladies dégénératives cérébrales, telles que les démences séniles, la maladie d'Alzheimer et les démences de type Alzheimer.

On sait que dans ces maladies, les neurones centraux cholinergiques sont les premiers atteints par la dégénérescence, que ce soit au niveau de la synthèse du neuro-médiateur : l'acétyl-choline, ou au niveau de la membrane qui nécessite de la choline pour sa biosynthèse. Or du point de vue thérapeutique, on ne peut pas administrer directement de l'acétyl-choline quine traverse pas les membranes, et les résultats obtenus par administration de choline seule sous forme de chlorure sont tout à fait insuffisants.

Le nouveau composé selon l'invention a fait l'objet d'un certain nombre d'expérimentations conduites sur deux modèles animaux de dégénérescence nerveuse, provoquée soit par une destruction des noyaux cholinergiques de la base, soit par l'administration réitérée d'hydroxyde d'aluminium. On a observé dans les deux cas, que les capacités d'apprentissage des animaux, détériorées en liaison avec la dégénérescence, se trouvaient restaurées par l'administration par voie orale du diéthyl-dithio-carbamate de choline selon l'invention. Après un traitement de trois semaines, on observait un effetdose et les mesures de dose efficace révélaient que la $DE_{50}$ était comprise entre 0,10 g et 0,30 g/kg de poids d'animal.

L'étude clinique de diéthyl-dithio-carbamate de choline a été réalisée en procédant à des expérimentations cliniques sur des patients atteints d'une maladie dégénérative cérébrale, parfaitement définie selon les critères DSM III du MMS, d'un bilan psychométrique approfondi et d'une radiographie SCANER.

L'expérimentation a été menée en double insu, en milieu hospitalier avec les traitements suivants d'une durée de six à douze semaines :

— placebo
— chlorure de choline
— diéthyl-dithio-carbamate de choline.

Les doses de substance active utilisées étaient de 125 mg par prise orale trois fois par jour, soit une dose journalière de 375 mg.

Les résultats montrent que dans 70% des cas traités par le produit selon l'invention, on observe une suppression ou une réduction significative des troubles de comportement et de l'humeur des patients, ces résultats

étant objectivés par des tests psychométriques.

Dans le cas des patients traités avec le placebo ou le chlorure de choline, on observe dans 30% des cas seulement des améliorations légères, peu nettes et moins durables que celles obtenues dans le cas des patients traités avec le nouveau composé de l'invention.

La présente invention a également pour objet des compositions thérapeutiques contenant le diéthyl-dithiocarbamate de choline à titre de principe actif. Ces compositions peuvent être administrées par voie orale, rectale, ou injectable, selon l'état physiopathologique du tractus digestif des malades considérés.

Dans le cas de l'administration orale, les compositions pharmaceutiques de l'invention se présentent sous forme de gélules ou comprimés dans lesquels le composé actif est associé à un excipient convenable, comprenant avantageusement du gel de silice pour tenir compte du caractère fortement hygroscopique du diéthyl-dithio-carbamate de choline. Par ailleurs pour éviter l'hydrolyse prématurée de ce dernier au niveau de l'estomac, il est nécessaire que l'enveloppe galénique de ces préparations pour administration orale soit de nature entérique ou gastro résistante, c'est-à-dire ne se délitant qu'en milieu intestinal (par exemple micro-encapsulation).

Dans le cas de l'administration par voie rectale, on peut préparer à partir du composé selon l'invention des suppositoires de composition classique.

Dans le cas de la voie injectable il est nécessaire de prévoir le conditionnement séparé du diéthyl-dithiocarbamate de choline et du soluté physiologique qui devra lui être additionné au moment de l'administration.

La posologie retenue pour les nouvelles compositions pharmaceutiques selon l'invention se situe entre une administration journalière de 250 mg à 2,5 g de composé actif, étant entendu que pour l'administration orale les gélules ou comprimés sont de préférence dosés à 125 mg du composé actif et peuvent être administrés à raison de 2 à 20 gélules ou comprimés par jour, et que pour l'administration rectale les suppositoires sont dosés à 250 ou 500 mg de composé actif pour 3 g.

## Revendications

### Revendications pour les Etats Contractants : AT, BE, CH, DE, FR, GB, IT, LI, LU, NL, SE

1. Le diéthyl-dithio-carbamate de choline ayant la formule développée suivante

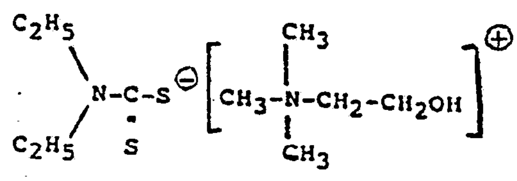

2. Procédé de préparation du diéthyl-dithio-carbamate de choline caractérisé en ce qu'on fait réagir du chlorure de choline sur du diéthyl-dithio-carbamate de sodium tri-hydraté.

3. Compositions thérapeutiques utiles dans le traitement des maladies dégénératives cérébrales, caractérisées en ce qu'elles contiennent à titre de composé actif du diéthyl-dithio-carbamate de choline.

4. Compositions thérapeutiques selon la revendication 3, caractérisées en ce qu'elles se présentent sous forme de gélules ou comprimés.

5. Compositions thérapeutiques selon la revendication, caractérisées en ce qu'elles se présentent sous forme de suppositoires.

6. Compositions thérapeutiques selon la revendication 4, caractérisées en ce que les gélules ou comprimés présentent un enrobage avec une substance gastro-résistante ne se délitant qu'en milieu intestinal.

7. Compositions thérapeutiques selon l'une quelconque des revendications 3, 5 et 6, caractérisées en ce que les gélules ou comprimés contiennent un excipient comportant du gel de silice.

8. Utilisation du diéthyl-dithio-carbamate de choline pour la fabrication d'un médicament destiné au traitement des malades dégénératives cérébrales.

### Revendications pour l'Etat Contractant : GR

1. Le diéthyl-dithio-carbamate de choline ayant la formule développée suivante

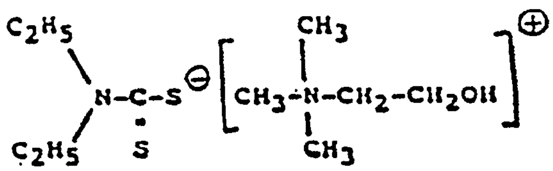

2. Procédé de préparation du diéthyl-dithio-carbamate de choline caractérisé en ce qu'on fait réagir du chlorure de choline sur du diéthyl-dithio-carbamate de sodium tri-hydraté.

3. Procédé de préparation d'une composition pharmaceutique utile dans le traitement des maladies dégénératives cérébrales, caractérisé en ce que l'on met du diéthyl-dithio-carbamate de choline sous une forme pharmaceutiquement acceptable.

4. Procédé selon la revendication 3, caractérisé en ce que l'on met le diéthyl-dithio-carbamate de choline sous forme de gélules ou comprimés.

5. Procédé selon la revendication 3, caractérisé en ce que l'on met le diéthyl-dithio-carbamate de choline sous forme de suppositoires.

6. Procédé selon la revendication 3, caractérisé en ce que l'on enrobe les gélules ou comprimés avec une substance gastro-résistante.

7. Procédé selon l'une quelconque des revendications 3, 5 et 6, caractérisé en ce que les gélules ou comprimés contiennent un excipient comportant du gel de silice.

8. Utilisation du diéthyl-dithio-carbamate de choline pour la fabrication d'un médicament destiné au traitement des maladies dégénératives cérébrales.

**Revendications pour l'Etat Contractant : ES**

1. Procédé de préparation du diéthyl-dithio-carbamate de choline de formule

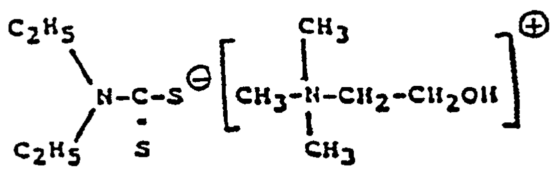

caractérisé en ce qu'on fait réagir du chlorure de choline sur du diéthyl-dithio-carbamate de sodium tri-hydraté.

2. Procédé de préparation d'une composition pharmaceutique utile dans le traitement des maladies dégénératives cérébrales, caractérisé en ce que l'on met du diéthyl-dithio-carbamate de choline sous une forme pharmaceutiquement acceptable.

3. Procédé selon la revendication 2, caractérisé en ce que l'on met le diéthyl-dithio-carbamate de choline sus forme de gélules ou comprimés.

4. Procédé selon la revendication 2, caractérisé en ce que l'on met le diéthyl-dithio-carbamate de choline sous forme de suppositoires.

5. Procédé selon la revendication 3, caractérisé en ce que l'on enrobe les gélules ou comprimés avec une substance gastro-résistante.

6. Procédé selon l'une quelconque des revendications 2, 4 et 6, caractérisé en ce que les gélules ou comprimés contiennent un excipient comportant du gel de silice.

7. Utilisation du diéthyl-dithio-carbamate de choline pour la fabrication d'un médicament destiné au traitement des maladies dégénératives cérébrales.

**Patentansprüche**

**Patentansprüche für folgenden Vertragsstaaten : AT, BE, CH, DE, FR, GB, IT, LI, LU, NL, SE**

1. Cholin-diethyl-dithio-carbamat der Formel

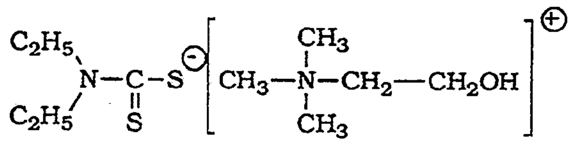

2. Verfahren zur Herstellung von Cholin-diethyl-dithio-carbamat, **dadurch gekennzeichnet,** daß man Cholinchlorid mit Natrium-diethyl-dithio-carbamat-Trihydrat umsetzt.

3. Pharmazeutische Zubereitungen zur Behandlung von cerebralen degenerativen Erkrankungen, **dadurch gekennzeichnet,** daß sie als Wirkstoff Cholin-diethyl-dithio-carbamat enthalten.

4. Pharmazeutische Zubereitungen nach Anspruch 3, **dadurch gekennzeichnet,** daß sie in Form von Gelkügelchen oder Tabletten vorliegen.

5. Pharmazeutische Zubereitungen nach Anspruch 3, **dadurch gekennzeichnet,** daß sie in Form von Suppositorien vorliegen.

6. Pharmazeutische Zubereitungen nach Anspruch 4, **dadurch gekennzeichnet,** daß die Gelkügelchen oder Tabletten mit einer gegenüber dem Magensaft resistenten Umhüllung versehen sind, die sich nur im Darmmedium auflöst.

7. Pharmazeutische Zubereitungen nach einem der Ansprüche 3, 5 und 6, **dadurch gekennzeichnet,** daß die Gelkügelchen oder Tabletten einen Kieselgel enthaltenden Träger enthalten.

8. Verwendung von Cholin-diethyl-dithio-carbamat zur Herstellung eines Arzneimittels zur Behandlung von cerebralen degenerativen Erkrankungen.

**Patentansprüche für folgenden Vertragsstaat : GR**

1. Cholin-diethyl-dithio-carbamat der Formel

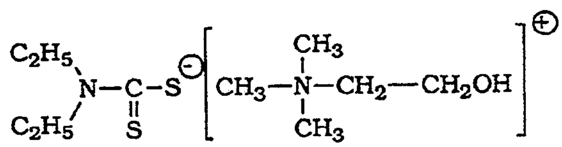

2. Verfahren zur Herstellung von Cholin-diethyl-dithio-carbamat, **dadurch gekennzeichnet,** daß man Cholinchlorid mit Natrium-diethyl-dithio-carbamat-Trihydrat umsetzt.

3. Verfahren zur Herstellung einer pharmazeutischen Zubereitung zur Behandlung von degenerativen cerebralen Erkrankungen, **dadurch gekennzeichnet,** daß man Cholin-diethyl-dithio-carbamat in eine pharmzeutisch annehmbare Form bringt.

4. Verfahren nach Anspruch 3, **dadurch gekennzeichnet,** daß man Cholin-diethyl-dithio-carbamat in die Form von Gelkügelchen oder Tabletten bringt.

5. Verfahren nach Anspruch 3, **dadurch gekennzeichnet,** daß man Cholin-diethyl-dithio-carbamat in die Form von Suppositorien bringt.

6. Verfahren nach Anspruch 3, **dadurch gekennzeichnet,** daß man die Gelkügelchen oder Tabletten mit einer gegenüber dem Magensaft resistenten Substanz umhüllt.

7. Verfahren nach einem der vorhergehenden Ansprüche 3, 5 und 6, **dadurch gekennzeichnet,** daß die Gelkügelchen oder Tabletten einen Kieselgel enthaltenden Träger enthalten.

8. Verwendung von Cholin-diethyl-dithio-carbamat zur Herstellung eines Arzneimittels zur Behandlung von degenerativen cerebralen Erkrankungen.

**Patentansprüche für folgenden Vertragsstaat : ES**

1. Verfahren zur Herstellung von Cholin-diethyl-dithio-carbamat der Formel

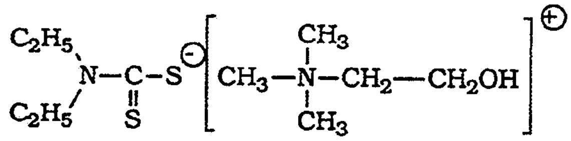

**dadurch gekennzeichnet,** daß man Cholinchlorid mit Natrium-diethyl-dithio-carbamat-Trihydrat umsetzt.

2. Verfahren zur Herstellung einer pharmazeutischen Zubereitung zur Behandlung von degenerativen cerebralen Erkrankungen, **dadurch gekennzeichnet,** daß man Cholin-diethyl-dithio-carbamat in eine pharmzeutisch annehmbare Form bringt.

3. Verfahren nach Anspruch 3, **dadurch gekennzeichnet,** daß man Cholin-diethyl-dithio-carbamat in die Form von Gelkügelchen oder Tabletten bringt.

4. Verfahren nach Anspruch 3, **dadurch gekennzeichnet,** daß man Cholin-diethyl-dithio-carbamat in die Form von Suppositorien bringt.

5. Verfahren nach Anspruch 3, **dadurch gekennzeichnet,** daß man die Gelkügelchen oder Tabletten mit einer gegenüber dem Magensaft resistenten Substanz umhüllt.

6. Verfahren nach einem der vorhergehenden Ansprüche 3, 5 und 6, **dadurch gekennzeichnet,** daß die Gelkügelchen oder Tabletten einen Kieselgel enthaltenden Träger enthalten.

7. Verwendung von Cholin-diethyl-dithio-carbamat zur Herstellung eines Arzneimittels zur Behandlung von degenerativen cerebralen Erkrankungen.

## Claims

### Claims for the following Contracting States : AT, BE, CH, DE, FR, GB, IT, LI, LU, NL, SE

1. Choline diethyldithiocarbamate having the following structural formula :

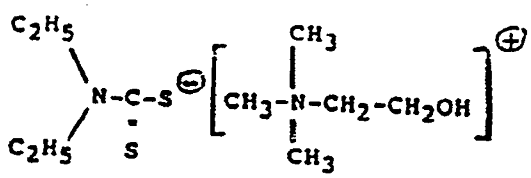

2. A process for the preparation of choline diethyldithiocarbamate, characterised in that choline chloride is reacted on trihydrated sodium diethyldithiocarbamate.

3. Therapeutic compositions of use in the treatment of degenerative cerebral diseases, characterised in that they contain choline diethyldithiocarbamate as the active compound.

4. Therapeutic compositions according to claim 3, characterised in that they are presented in the form of capsules or tablets.

5. Therapeutic compositions according to claim 3, characterised in that they are presented in the form of suppositories.

6. Therapeutic compositions according to claim 4, characterised in that the capsules or tablets are coated with a gastro-resistant substance which disintegrates only in the intestinal medium.

7. Therapeutic compositions according to any one of claims 3, 5 and 6, characterised in that the capsules or tablets contain an excipient comprising silica gel.

8. Use of choline diethyldithiocarbamate for the production of a drug intended For the treatment of degenerative cerebral diseases.

### Claims for the following Contracting State : GR

1. Choline diethyldithiocarbamate having the following structural formula :

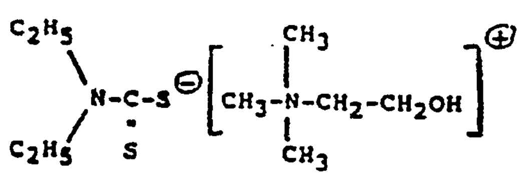

2. A process for the preparation of choline diethyldithiocarbamate, characterised in that choline chloride is reacted on trihydrated sodium diethyldithiocarbamate.

3. A process for the preparation of a pharmaceutical composition of use in the treatment of degenerative

cerebral diseases, characterised in that choline diethyldithiocarbamate is put into a pharmaceutically acceptable form.

4. A process according to claim 3, characterised in that choline diethyldithiocarbamate is put into the form of capsules or tablets.

5. A process according to claim 3, characterised in that choline diethyldithiocarbamate is put into the form of suppositories.

6. A process according to claim 3, characterised in that the capsules or tablets are coated with a gastro-resistant substance.

7. A process according to any one of claims 3, 5 and 6, characterised in that the capsules or tablets contain an excipient comprising silica gel.

8. Use of choline diethyldithiocarbamate for the production of a drug intended for the treatment of degenerative cerebral diseases.

**Claims for the following Contracting State : ES**

1. A process for the preparation of choline diethylithiocarbamate having the following structural formula :

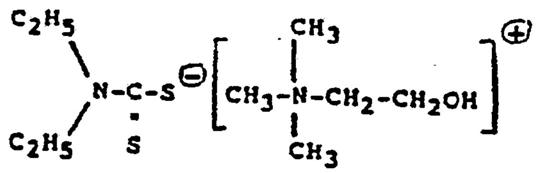

characterised in that choline chloride is reacted on trihydrated sodium diethyldithiocarbamate.

2. A process for the preparation of a pharmaceutical composition of use in the treatment of degenerative cerebral diseases, characterised in that choline diethyldithiocarbamate is put into a pharmaceutically acceptable form.

3. A process according to claim 3, characterised in that choline diethyldithiocarbamate is put into the form of capsules or tablets.

4. A process according to claim 3, characterised in that choline diethyldithiocarbamate is put into the form of suppositories.

5. A process according to claim 3, characterised in that the capsules or tablets are coated with a gastro-resistant substance.

6. A process according to any one of claims 2, 4 and 6, characterised in that the capsules or tablets contain an excipient comprising silica gel.

7. Use of choline diethyldithiocarbamate for the production of a drug intended for the treatment of degenerative cerebral diseases.